# EUROPEAN PATENT SPECIFICATION

(11) **EP 3 886 942 B1**
(45) Date of publication and mention of the grant of the patent: **27.08.2025**
(21) Application number: 18941565.6
(22) Date of filing: 29.11.2018
(51) Int. Cl.: A61M 5/00, A61M 5/34, A61M 5/20, A61M 5/178

(54) **PEN AND PEN NEEDLE ADAPTER WITH FEATURE TO IMPROVE ACCESS TO PACKAGED NEEDLE ASSEMBLY**
STIFT UND NADELADAPTER FÜR EINEN STIFT MIT VERBESSERTEM ZUGANG ZU EINER VERPACKTEN NADELANORDNUNG
STYLO ET ADAPTATEUR D'AIGUILLE DE STYLO AYANT UNE CARACTÉRISTIQUE POUR AMÉLIORER L'ACCÈS À UN ENSEMBLE AIGUILLE EMBALLÉ

(43) Date of publication of application: 06.10.2021
(73) Proprietor: Embecta Corp., Andover, MA 01810 (US)
(72) Inventor: WEST, Robert, Basking Ridge, New Jersey 07920 (US)
(74) Representative: dompatent von Kreisler Selting Werner - Partnerschaft von Patent- und Rechtsanwälten mbB
(86) International application number: PCT/US2018/063047
(87) International publication number: WO 2020/112113

(56) References cited:
- US-A- 5 829 589
- US-A1- 2001 014 792
- US-A1- 2001 014 792
- US-A1- 2011 106 016
- US-A1- 2012 130 342
- US-A1- 2012 130 342
- US-A1- 2015 025 473
- US-A1- 2015 025 473
- US-A1- 2016 207 684

## Description

### BACKGROUND

### Field:

Illustrative embodiments relate generally to a pen or pen needle adapter configured to access packaged pen needles for medication delivery pens and, more particularly, to a tip or distal end of a pen, or an adapter for a pen, that attaches to pen needles and that has a scything and capture feature to break and collect barrier material used on pen needle assembly packaging.

### Description of Related Art:

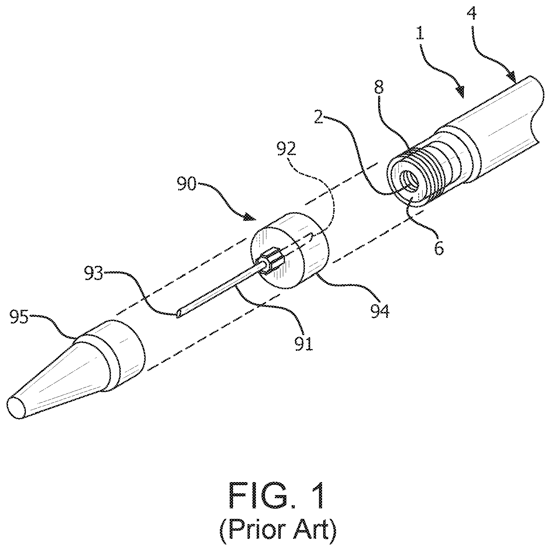

Medication delivery pens have been developed to facilitate the self-administration of medication. A conventional medication delivery pen is identified generally by the numeral 1 in FIG. 1. Pen 1 contains a cartridge with sufficient medication for several doses. The conventional cartridge has opposed proximal and distal ends. The distal end is closed by a pierceable and resealable rubber septum identified by the numeral 2 in FIG. 1. The proximal end receives a stopper in sliding fluid-tight engagement. The conventional cartridge is disposed in an elongate pen-like body 4 with a proximal end (not shown) and an opposed distal end 6. The proximal end of the pen body includes a plunger for selectively driving the stopper of the cartridge in the distal direction and a dose setting mechanism for determining the distance through which the plunger and stopper can move. Distal end 6 of pen body 4 includes an array of threads 8 for threaded engagement with a pen needle assembly 90. Pen needle assembly 90 includes a needle cannula 91 with opposed proximal and distal points 92 and 93 and a threaded mounting skirt 94 which surrounds the proximal tip 92. Mounting skirt 94 is threadably engageable with threads 8 on distal end 6 of pen body 4. A safety shield 95 is releasably engaged over distal point 93 and portions of mounting skirt 94 to prevent accidental needle sticks. Existing medication delivery pens are further disclosed in U.S. Patent Application Publication Nos. 2001/014792A1 to R. West.

A person who must periodically inject doses of medication will carry a medication delivery pen 1 and a supply of pen needle assemblies 90. Each pen needle assembly 90 has its needle cannula 91 safely and sterility sealed in its own shield 95, and is accessed immediately prior to administering a dose of medication. Pen needle assembly 90 is then mounted to distal end 6 of the pen 1. This mounting causes proximal point 92 of needle cannula 91 to pierce rubber septum 2 of the cartridge, to place needle cannula 91 in communication with the medication in pen 1. Pen 1 is then used to inject the selected dose of medication. After completing the injection, needle assembly 90 is separated from pen 1 and is discarded. Pen 1 may be used repeatedly in this manner until the medication is exhausted. Such pens 1 offer many conveniences and efficiencies. However, the storage of unused needles and the final disposal of used needles has presented problems. In particular, supplies of new needles often are loosely scattered in the bottom of purses or briefcases, and used needles are often disposed of unsafely.

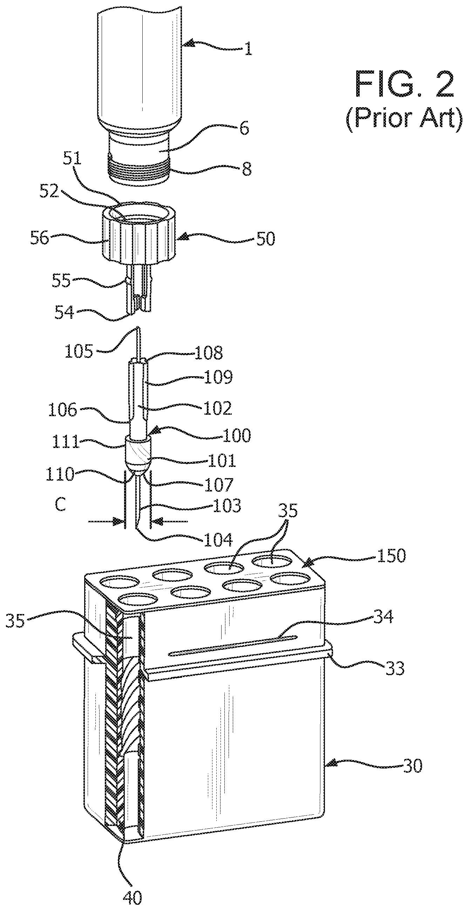

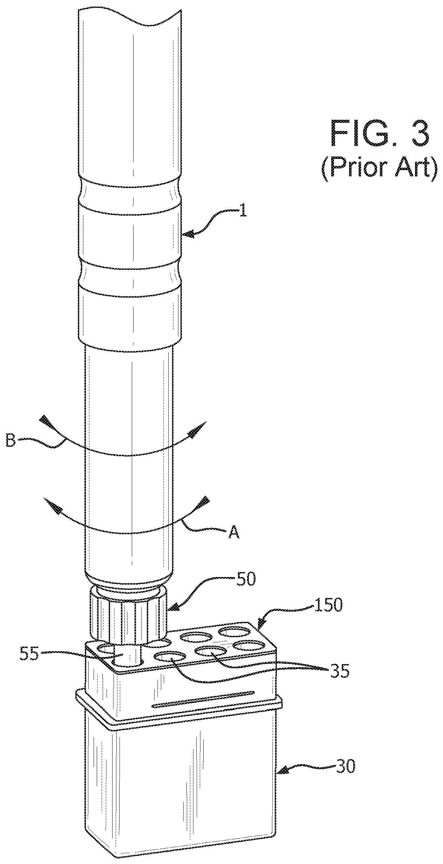

With reference to FIGs. 2 and 3, a pen needle magazine dispenser is configured to store sterile, unused needles and provides means to store used needles until final disposal. As shown in FIG. 2, an example pen needle magazine dispenser includes a container 30 which can have a cover (not shown). Container 30 includes a top surface covered by a sterility barrier 150. Container 30 includes a plurality of cavities 35, with each cavity 35 dimensioned to receive a sleeve 40 containing a pen needle assembly 100, described further below. Each pen needle assembly 100 is originally sealed in its respective sleeve 40 and cavity 35 by the sterility barrier 150 that is attached to the top surface of container 30. The sterility barrier 150 can comprise, for example, a substrate with apertures aligned with the cavities 35 and a clear or opaque plastic or foil film adhered to the substrate. The sterility barrier 150 can also be adhered directly to the top surface of the container 30. Thus, the sterility barrier 150 provides sterility for unused pen needle assemblies 100 contained in each sleeve 40 in a cavity 35, and a simple means for the user to identify whether the pen needle assembly 100 in a particular sleeve 40 has been used. For example, the sterility barrier 150 can have indicia printed thereon such as indicia printed over the aperture for each cavity 35 that corresponds to a location of a pen needle assembly 100.

FIG. 2 also depicts an exploded perspective view of a pen needle assembly 100, an adapter 50 and a medication delivery pen 1 exploded therefrom. Adapter 50 includes a hub 56 with an open proximal end 51 having a set of threads 52 dimensioned to mate with threads 8 on distal end 6 of conventional medication delivery pen 1. Adapter 50 also includes a post 55 with an opening 53 in its distal end 54. The hub 56 and post 55 share a channel for receiving a needle assembly 100. For example, the hub 56 and post 55 can be defined by a tubular wall and are integral to the adapter. After adapter 50 has been threaded onto distal end 6 of pen 1, distal end 54 of adapter 50 is used to remove pen needle assembly 100 from sleeve 40 by inserting key 109 on proximal end 108 of needle assembly 100 into a corresponding key way within the adapter 50.

With reference to FIG. 2, pen needle assembly 100 includes a hub 102 having a plurality of keys 109 and a distal end 107 having a set of threads 101 thereon dimensioned to mate with the set of threads 44 within sleeve 40. A needle cannula 103 is mounted within hub 102 and includes a distal point 104 and a proximal point 105. When a key 109 of needle assembly 100 is inserted into a corresponding key way in the adapter 50, rotation of medication delivery pen 1 causes pen needle assembly 100 to rotate within sleeve 40 and move key 109 of needle assembly 100 along the key way into a channel in the adapter 50. As key 109 moves from the key way into the channel of the adapted 50, edge 106 moves over a helical surface in the adapter 50 so to lock key 109 in the adapter channel and prevent pen needle assembly 100 from sliding out of adapter 50. The adapter's helical surface interaction with edge 106 also aids in driving pen needle assembly 100 into adapter 50 to a predetermined position where surface 111 is in contact with distal end 54 of adapter 50 to firmly hold pen needle assembly 100 within adapter 50.

To remove an unused needle assembly 100 from a cavity 35 of the container 30, a pen 1 with adapter 50 is inserted into a cavity 35 in the container 30 of the pen needle magazine dispenser, as shown in FIG. 3, and rotated (e.g., in the clockwise direction A) to attach a needle assembly 100 in the cavity 35 to the adapter 50 as described above. After use, the used pen needle assembly 100 mounted on adapter 50 on medication delivery pen 1 is reinserted into the sleeve 40 in the cavity until detent 55 and retention groove 43 mate and the set of threads 101 on pen needle assembly 100 come into contact with the set of threads 44 within sleeve 40. Medication delivery pen 1 is then rotated in the opposite direction B, shown in FIG. 3, to thread pen needle assembly 100 back into sleeve 40 and pen 1 is then pulled out of container 30 as pen needle assembly 100 is pulled out of adapter 50.

The distal end 54 of the post 55 of the adapter 50 must break through the sterility barrier 150 over the cavity 35 to access the needle assembly 100 inside. A problem occurs when a piece(s) of the broken barrier 150 is pushed into the cavity 35 or otherwise bunches at the opening to the cavity 35, which can impede connection between the adapter 50 and the pen needle assembly 100 and/or impede movement of the connected adapter and needle assembly in and out of the cavity 35.

### SUMMARY

The above and other problems are overcome, and additional advantages are realized, by an injection pen having the features defined within claim 1 or by an adapter for an injection pen having the features defined within claim 4.

It is an aspect of illustrative embodiments to provide a pen body, or detachable pen needle adapter, for an injection pen that comprises a tubular wall having a proximal end, and an opposite distal end configured to be connected to a needle assembly; wherein the distal end of the tubular wall comprises a through cut, the through cut defining an opening in the tubular wall, the through cut having an edge configured to pierce barrier material provided on needle assembly packaging when the distal end is depressed against the needle assembly packaging to access a needle assembly stored therein, and the opening of the through cut being configured to receive barrier material after it has been pierced to guide it away from the needle assembly.

In accordance with aspects of illustrative embodiments, the edge of the through cut comprises a tooth that extends from the distal end.

In accordance with aspects of illustrative embodiments, the tooth extends from the distal end by a distance corresponding to at least the thickness of the barrier material.

In accordance with aspects of illustrative embodiments, the tooth comprises a pointed tip or a rounded tip.

In accordance with aspects of illustrative embodiments, the edge of the through cut comprises a scything edge along at least one side of the opening.

In accordance with aspects of illustrative embodiments, the opening of the through cut has an arcuate shape, or a straight shape.

In accordance with aspects of illustrative embodiments, the through cut is disposed in the tubular wall at a designated angle between a longitudinal axis of the pen body and a perpendicular axis at the distal end. For example, the designated angle is selected to capture the barrier material after being pierced by the edge of the through cut and guide the barrier material away from the needle assembly.

In accordance with aspects of illustrative embodiments, the through cut has a distal end adjacent the distal end of the pen body and a proximal end that is opposite the distal end of the through cut, and the opening extends between the distal end of the through cut and the proximal end of the through cut; and wherein the through cut is arranged in a selected one of a first direction and a second direction, the first direction allowing the distal end of the through cut to precede the proximal end of the through cut when the pen is rotated in a clockwise direction about its longitudinal axis, and the second direction allowing the distal end of the through cut to precede the proximal end of the through cut when the pen is rotated in a counterclockwise direction about its longitudinal axis.

In accordance with aspects of illustrative embodiments, the distal end is dimensioned to fit in a cavity of the needle assembly packaging in which a needle assembly is stored and to receive at least part of the needle assembly.

It is an aspect of illustrative embodiments to provide an adapter for an injection pen having a hub comprising a tubular wall having a proximal end configured to be connected to an injection pen and the distal end thereof configured to be connected to a needle assembly, the tubular wall defining a channel therein that extends between the proximal end and the distal end and along a longitudinal axis of the hub, the channel being configured to receive at least part of a needle assembly therein; and wherein the distal end of the tubular wall comprises the through cut

In accordance with aspects of illustrative embodiments, the hub is dimensioned at its proximal end to mate with a pen. Further, the hub has a post section at its distal end that is dimensioned to fit in a cavity of the needle assembly packaging in which a needle assembly is stored and to receive at least part of the needle assembly; and the through cut is disposed in the post section of the hub.

Additional and/or other aspects and advantages of the present invention will be set forth in the description that follows, or will be apparent from the description, or may be learned by practice of the invention. The present invention may comprise pen needle adapters and methods for operating same having one or more of the above aspects, and/or one or more of the features and combinations thereof. The present invention may comprise one or more of the features and/or combinations of the above aspects as recited, for example, in the attached claims.

### BRIEF DESCRIPTION OF THE DRAWINGS

The above and/or other aspects and advantages of embodiments of the invention will be more readily appreciated from the following detailed description, taken in conjunction with the accompanying drawings, of which:
FIG. 1 is an exploded perspective view of a conventional pen needle and the distal end of a conventional medication delivery pen;
FIG. 2 is an exploded perspective view of a conventional pen, a conventional pen needle and a conventional partially assembled pen needle magazine dispenser;
FIG. 3 is a perspective view of the medication delivery pen and pen needle magazine dispenser of FIG. 2, when attaching or removing the pen needle from the adapter;
FIG. 4A is a perspective view of an adapter constructed in accordance with an illustrative embodiment for use with a medication delivery pen to load a pen needle assembly;
FIGs. 4B and 4C are, respectively, partial perspective and cross-section views of the adapter in FIG. 4A;
FIGs. 5A, 5B and 5C are a sequence of views showing an adapter constructed in accordance with an illustrative embodiment piercing and scything a barrier material covering a cavity containing a pen needle assembly, and capturing and moving the material away from the cavity opening;
FIG. 6A is a perspective view of an adapter constructed in accordance with another illustrative embodiment for use with a medication delivery pen to load a pen needle assembly;
FIGs. 6B and 6C are, respectively, partial perspective and cross-section views of the adapter in FIG. 6A;
FIG. 7A is a perspective view of an adapter constructed in accordance with another illustrative embodiment for use with a medication delivery pen to load a pen needle assembly; and

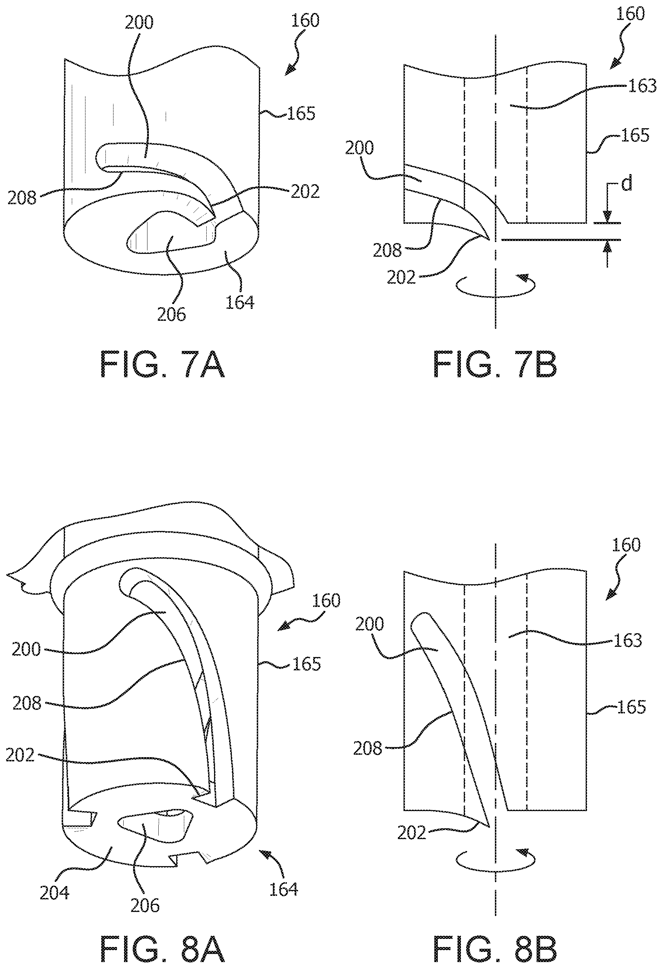

FIGs. 7A and 7B are, respectively, partial perspective and cross-section views of an adapter constructed in accordance with yet another illustrative embodiment for use with a medication delivery pen to load a pen needle assembly;
FIGs. 8A and 8B are, respectively, partial perspective and cross-section views of an adapter constructed in accordance with still yet another illustrative embodiment for use with a medication delivery pen to load a pen needle assembly;

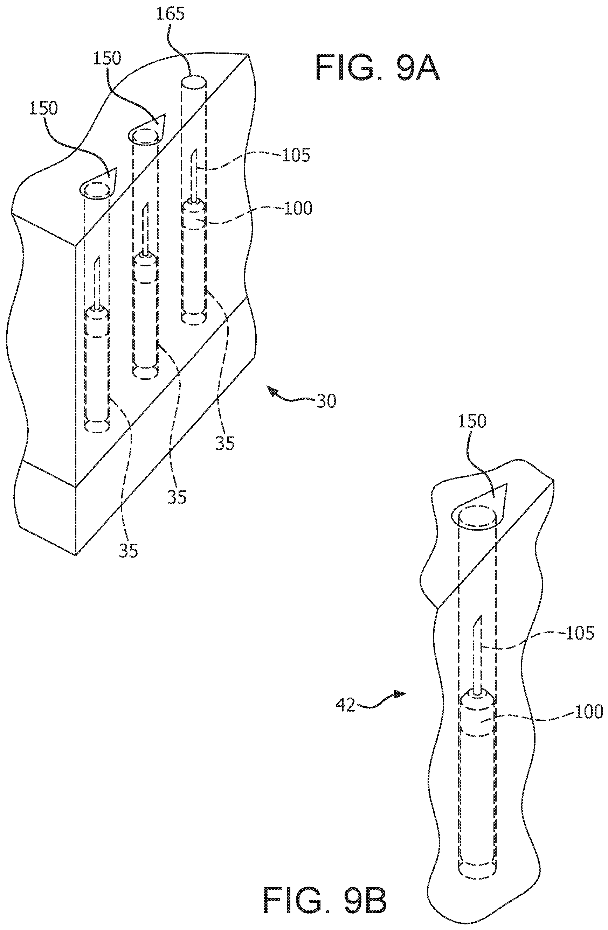

FIG. 9A is a perspective view of an example pen needle magazine dispenser having an individual sterility barrier for a corresponding cavity containing a pen needle assembly; and
FIGs. 9A and 9B are perspective views, respectively, of an example needle assembly container, and an example package having a pen needle assembly and an individual sterility barrier.

Throughout the drawing figures, like reference numbers will be understood to refer to like elements, features and structures.

### DETAILED DESCRIPTION OF ILLUSTRATIVE EMBODIMENTS

Reference will now be made in detail to embodiments of the present invention, which are illustrated in the accompanying drawings. The embodiments described herein exemplify, but do not limit, the present invention by referring to the drawings.

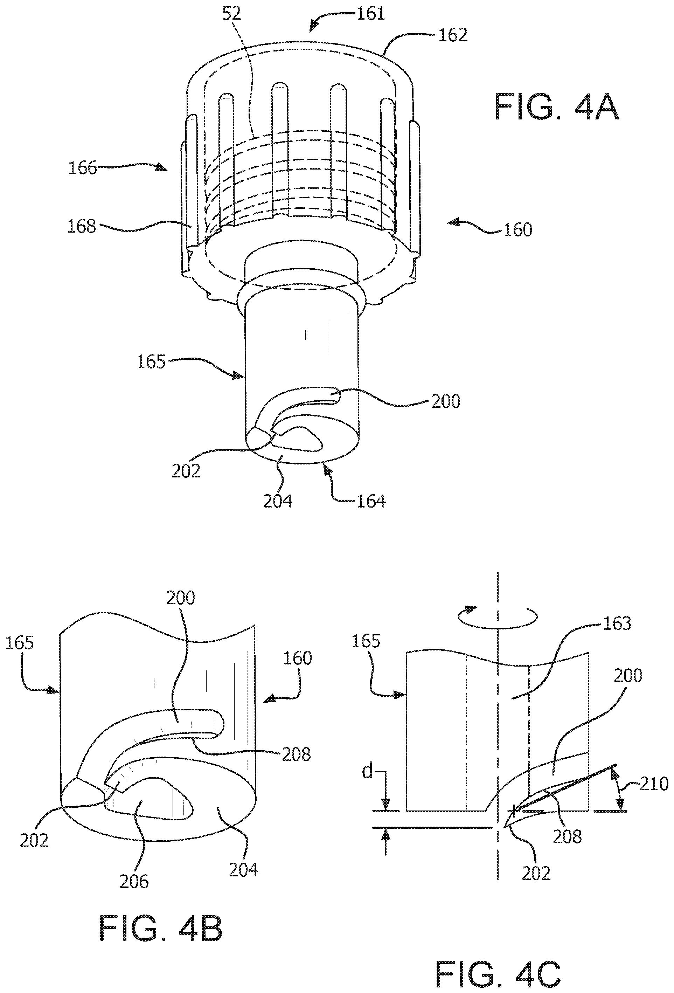

With reference to FIGs. 4A, 4B and 4C, the tip or distal end of an example pen, or an example detachable adapter 160 for the pen, is constructed in accordance with an illustrative embodiment to overcome the above-described problems of a sterility barrier or other material 150 covering a cavity 35 in the container 30 of a pen needle magazine dispenser or covering a package (e.g. the package 42 in Fig. 9B containing a single needle assembly 100). As described herein, the tip or distal end 164 of an example adapter 160 shall be described in accordance with different example embodiments illustrated in FIGs. 4A through 8B. It is to be understood, however, that the tip or distal end 164 of the adapter 160 can be integrally provided to an example pen. In other words, the adapter 160 is not threaded or snap-fit to the end of a pen, but rather the pen has a distal end or tip thereof that is integrally formed thereon and similar to the distal end or tip 164 of the adapter 160 in accordance with the different example embodiments illustrated in FIGs. 4A through 8B.

When pierced to remove a needle assembly 100, all or part of the barrier or packaging material 150 can bunch at the opening to the cavity 35 or package 42, which can impede connection between the adapter 160 and the needle assembly 100 and/or impede movement of the connected adapter and needle assembly in and out of the cavity 35 or package 42. It is to be understood that the sterility barrier or other material 150 used to enclose a pen needle assembly 100 (i.e., in a cavity 35 of a container 30 or an individual needle assembly package 42) has a designated thickness Th and can be deployed as a layer across multiple cavities in a container 35 (e.g., FIG. 2), or as a single label or sticker 150 over a package 42 (FIG. 9B) or over respective ones of the cavities 35 in a container 30 (FIG. 9A).

As shown in FIGs. 4A, 4B and 4C, the example adapter 160 has an interior space or channel 163 extending along its longitudinal axis that is dimensioned and shaped to receive a pen needle assembly 100 at its distal end 164. The adapter 160 has a proximal end 161 configured to be connected to or mounted on the distal end 6 of a pen 1. For example, the adapter 160 can have a hub 166 with threads 162 at its proximal end 161 for cooperating with threads 8 on the distal end 6 of a pen 1. The hub 166 can have an integral section thereof configured as a post 165 that is dimensioned at its distal end 164 to be inserted at least partially into a cavity 35 of a container, or into a package 42, that is dimensioned to store a needle assembly 100 therein. The hub 166 therefore can have a similar diameter to a pen 1 (e.g., about 3/8 inch (in) or 88 millimeters (mm) interior diameter of the interior space or channel 163 plus the thickness of the wall of the hub 166 on each side of the interior diameter). The hub 166 can be removably engaged with the pen 1 (e.g., via threads, or snap or pressure fit) or can be integral to the pen 1. The post 165 can have a diameter similar to that of a needle assembly 100 (e.g., about 1/8 in or less than 0.150 in as illustrated by an example diameter C shown in FIG. 2), for example. The shape of the interior space or channel 163 and the exterior shape of the hub 166 and post 165 are illustrated as tubular or cylindrical. It is to be understood that the interior space or channel 163, and the exterior shape of the hub 166 or post 165 can have different shapes such as triangular in cross-section of the interior space, or triangular perimeter of the hub 166 and/or post 165, and that the dimensions and shape can vary internally or externally along the longitudinal axis of the adapter 160. In addition, the exterior of the hub 160 can be provided with ribs 168 or other texture to improve gripping thereof by a user, or can be smooth.

In accordance with an advantageous aspect of illustrative embodiments, the tip or distal end 164 of the adapter 160 is provided with a through cut 200 in the tubular wall of the post 165. As will be described below in connection with various illustrative embodiments, the through cut 200 can have different lengths, widths, shapes and angles between a scything edge 208 of the through cut 200 and the distal end surface 204 of the adapter (hereinafter referred to as the scything angle 210), and each of these characteristics can impact the ability of the adapter to pierce and guide the barrier material 150 away from the cavity 35 or package 42 containing the needle assembly 100. In addition and accordance with another advantageous aspect of illustrative embodiments, the tip of the scything edge 208 can be configured as a tooth 202 with a shape (e.g. point) that facilitates piercing the material 150. Further, the tooth 202 can extend a selected distance from the distal end surface 204 to improve effectiveness of piercing the material 150, as well as catching the material 150 and guiding it away from the interior or opening of the cavity 35 or package 42.

In accordance with an illustrative embodiment depicted in FIGs. 4A, 4B and 4C, the through cut 200 has an arcuate shape. The length of the through cut 200, the width of the through cut 200, the degree of curve of the through cut 200's arcuate shape, and the direction of the through cut 200 (e.g., extending from the distal end surface 204 to the right or left direction such as to the right as depicted in the elevation view of the adapter 160 in FIG. 4C),and the scything angle 210 can be selected and can vary, depending on the flexibility and thickness of the material 150. For example, a larger width and/or length of the through cut 200 can allow capture of more material 150 than a smaller width and/or length. This larger width and/or length of the through cut 200 can be helpful when piercing a material 150 layer (e.g., a layer covering an area on top of the container 30 in FIG. 2 that includes plural cavity 35 openings) versus a material 150 label (e.g., a label covering only an individual cavity opening as shown in FIG. 9A). Further, the scything angle 210 and the degree of curve of the through cut 200's arcuate shape impacts the scything edge 208's ability to cut through the material and therefore both can be selected to optimize guidance of pierced and/or cut portion(s) 152 of the material 150 away from the interior or opening of the cavity 35 or package 42.

In accordance with another aspect of illustrated embodiments, the adapter 160 through cut 200 can be configured to form a tooth 202 that extends a selected distance from the distal end surface 204 to improve effectiveness of piercing the material 150. For example, the tooth 202 can extend beyond the distal end surface 204 by a distance d that is greater than or equal to the thickness of the material 150 (d ≥ Th) to aid piercing and scything. In addition, the tooth 202 can have different shapes such as having a pointed end, a rounded end, or a blunt end.

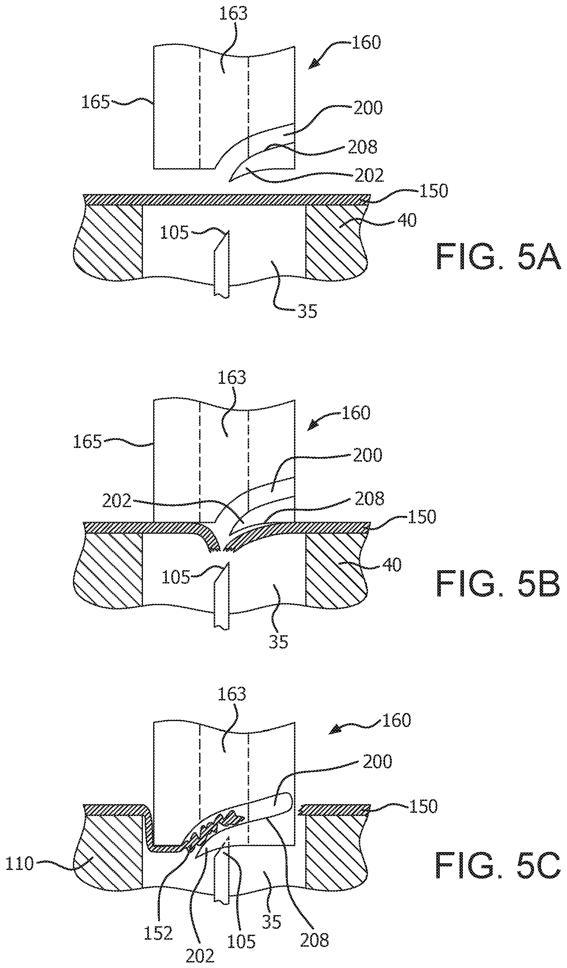

Reference is now made to FIGs. 5A, 5B and 5C, which depict a sequence of operations of the adapter 160 depicted in FIGs. 4A-4C piercing and scything a material 150 in accordance with an illustrative embodiment. For illustrative purposes, FIGs. 5A, 5B and 5C depict a partial, cross-sectional view of a layer of a material 150 covering a cavity 35 in a container 40 having a needle assembly disposed therein, as evidenced by the showing of the end of the needle assembly catheter 105 that is inserted into the space 163 in the adapter 160 when the adapter 160 successfully connects with the needle assembly 100. FIGs. 5A, 5B and 5C also depict a partial, cross-sectional of an adapter 160's post 165 having a through cut 200. Also shown are the scything edge 208 and tooth 202 of the adapter 160. In the illustrated embodiment of FIG. 5A, the tooth 202 of the adapter 160 is in contact with the material 150 but has not yet pierced it. When the adapter 160 is pressed by a user into the container cavity 35, which could also be another type of needle assembly package 42 with barrier material 150, the tooth 202 precedes the distal end surface 204 of the adapter 160 and pierces the material 150 to create a punctured opening in the material, as depicted in FIG. 5B. With reference to FIG. 5C, further movement of the post 165 down into the cavity 35 causes trimmed or cut material piece(s) 152 to be guided along the scything edge 208. Further, the adapter 160 can be rotated clockwise to encourage scything or cutting of the material 150 in a circular motion such that additional trimmed or cutaway material 152 can be guided away from the opening of the cavity by the guiding edge 208 of the through cut 200. Accordingly, the adapter 160 realizes the advantages of conveniently piercing the material 150 to access a stored, unused needle assembly 100 from a package (i.e., cavity 35 in a container 40 or interior space of a package 42), and ensuring that material pieces 152 are guided away from the storage cavity or space and therefore do not become lodged therein. The material 150 is therefore less likely to impede the connection of the adapter 160 (i.e., or pen with integrally formed with a tip or distal end that is similar to the distal end 164 of the adapter 160) to the stored needle assembly 100. Further, the material 152 is less likely to hinder movement of the connected needle assembly 100 from the cavity or space for use, and then back into the cavity or space for final storage and disposal after use.

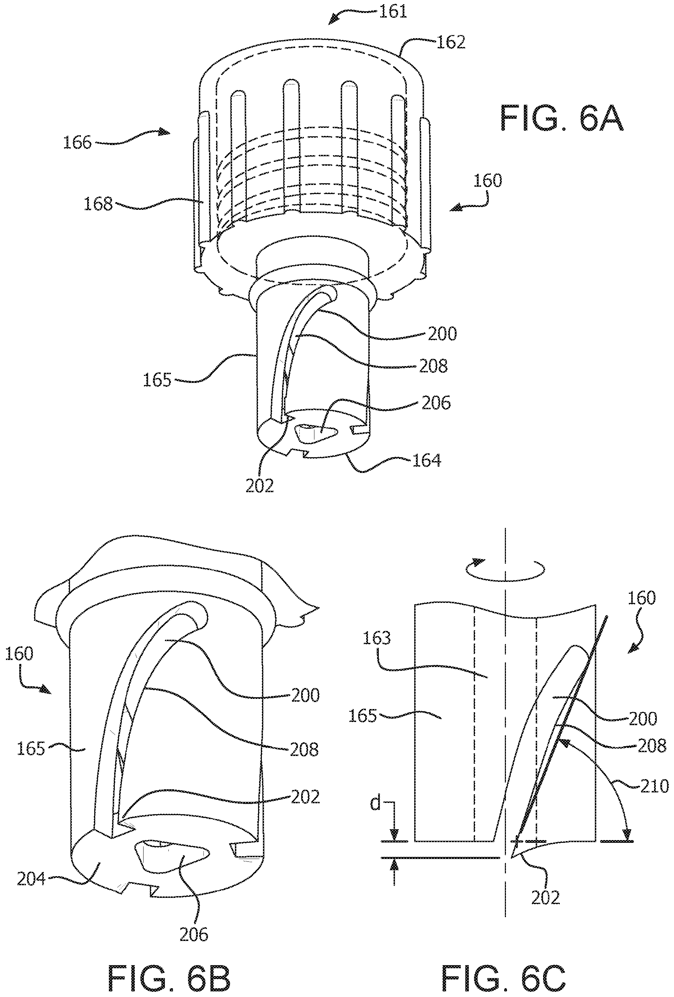

In accordance with another illustrative embodiment depicted in FIGs. 6A, 6B and 6C, an adapter 160 has a through cut 202 that is straight compared to the arcuate through cut in the adapter 160 depicted in FIGs. 4A, 4B and 4C. The adapter 160 can optionally have multiple through cuts 202 such as the three through cuts 202 disposed about the periphery of the tubular wall, as shown in Figs. 6A and 6B. Also, the scything angle 210 of the adapter 160 in FIGs. 6A, 6B and 6C is greater than the scything angle 210 of the adapter 160 depicted in FIGs. 4A, 4B and 4C. The straightened scything edge 208 and larger scything angle in FIGs. 6A, 6B and 6C can be more effective at physically moving pierced material 152 away from opening to cavity 35 or package 42 than the scything edge 208 and scything angle 210 of the adapter 160 depicted in FIGs. 4A, 4B and 4C. For example, the through cut 202 in FIGs. 6A, 6B and 6C can be dimensioned to have more space for capturing trimmed material piece(s) 152 that the through cut 202 in the adapter 160 depicted in FIGs. 4A, 4B and 4C. Also, the larger the scything angle 210 of the adapter 160 in FIGs. 6A, 6B and 6C can be useful to draw the material piece(s) 152 farther above and away from the container 40's cavity 35 or the interior space of a package 42 than the through cut 202 in the adapter 160 depicted in FIGs. 4A, 4B and 4C.

In accordance with another aspect of illustrated embodiments, the through cut 202 of an adapter 160 or distal end of a pen may not be completely through the wall of the post 165, such that distal end surface 204 can have solid perimeter around access orifice 206 as in FIG. 6B. As stated above, the hub 166 and/or port 165, and/or their opening 163 and access orifice 206, can be different cross-sectional shapes such as circular or triangular or other shape.

As stated above, the direction of the through cut 202 can be selected for operating the scything edge 208 during a clockwise or counterclockwise motion of the adapter 160 by a user. For example, the direction of the through cut 200 shown in FIGs. 4B and 4C is for clockwise rotation of the pen 1 with adapter 160 to pierce and cut barrier material 150 covering a container 40 or package 42. FIGs. 7A and 7B depict the through cut 200 shown in FIGs. 4B and 4C, except in an opposite direction for counterclockwise rotation of the pen 1 with adapter 160 to pierce and cut barrier material 150 covering a container 40 or package 42.

Similarly, the direction of the through cut 200 shown in FIGs. 6B and 6C is for clockwise rotation of the pen 1 with adapter 160 to pierce and cut barrier material 150 covering a container 40 or package 42. FIGs. 8A and 8B depict the through cut 200 shown in FIGs. 6B and 6C, except in an opposite direction for counterclockwise rotation of the pen 1 with adapter 160 to pierce and cut barrier material 150 covering a container 40 or package 42.

It will be understood by one skilled in the art that this disclosure is not limited in its application to the details of construction and the arrangement of components set forth in the above description or illustrated in the drawings. The embodiments herein are capable of other embodiments, and capable of being practiced or carried out in various ways. Also, it will be understood that the phraseology and terminology used herein is for the purpose of description and should not be regarded as limiting. The use of "including," "comprising," or "having" and variations thereof herein is meant to encompass the items listed thereafter and equivalents thereof as well as additional items. Unless limited otherwise, the terms "connected," "coupled," and "mounted," and variations thereof herein are used broadly and encompass direct and indirect connections, couplings, and mountings. In addition, the terms "connected" and "coupled" and variations thereof are not restricted to physical or mechanical connections or couplings. Further, terms such as up, down, bottom, and top are relative, and are employed to aid illustration, but are not limiting.

The above-presented description and figures are intended by way of example only and are not intended to limit the present invention in any way except as set forth in the following claims. It is particularly noted that persons skilled in the art can readily combine the various technical aspects of the various elements of the various illustrative embodiments that have been described above in numerous other ways, all of which are considered to be within the scope of the invention.

## Claims

1. An injection pen, the injection pen comprising:
a pen body (4) comprising a tubular wall having a proximal end (51), and an opposite distal end (54) configured to be connected to a needle assembly (100) of the injection pen;
wherein the distal end (54) of the tubular wall comprises a through cut (200), the through cut (200) defining an opening in the tubular wall, the through cut (200) having an edge configured to pierce a barrier material (150) provided on a needle assembly packaging (42) of the needle assembly (100) when the distal end (54) is depressed against the needle assembly packaging (42) to access the needle assembly (100) stored therein, and the opening of the through cut (200) being configured to receive the pierced barrier material (152) to guide the pierced barrier material (152) away from the needle assembly (100),
wherein the through cut (200) is disposed in the tubular wall at a designated angle (210) between a longitudinal axis of the pen body (4) and a perpendicular axis at the distal end (54);
wherein the designated angle (210) is selected so that the through cut captures the barrier material (150) after being pierced by the edge of the through cut (200) and guide the captured barrier material (150) away from the needle assembly (100).

2. The injection pen of claim 1, wherein the through cut (200) has a distal end adjacent the distal end (54) of the pen body (4) and a proximal end that is opposite the distal end of the through cut (200), and the opening extends between the distal end of the through cut (200) and the proximal end of the through cut (200); and
wherein the through cut (200) is arranged in a selected one of a first direction and a second direction, the first direction allowing the distal end of the through cut (200) to precede the proximal end of the through cut (200) when the pen is rotated in a clockwise direction about its longitudinal axis, and the second direction allowing the distal end of the through cut to precede the proximal end of the through cut (200) when the pen is rotated in a counterclockwise direction about its longitudinal axis.

3. The injection pen of claim 1, wherein the distal end (54) is dimensioned to fit in a cavity (35) of the needle assembly packaging (42) in which a needle assembly (100) is stored and to receive at least part of the needle assembly (100).

4. An adapter for an injection pen, the adapter comprising:
a hub comprising a tubular wall having a proximal end (51) configured to be connected to an injection pen and a distal end (54) configured to be connected to a needle assembly (100), the tubular wall defining a channel therein that extends between the proximal end (51) and the distal end (54) and along a longitudinal axis of the hub, the channel being configured to receive at least part of a needle assembly (100) therein;
wherein the distal end (54) of the tubular wall comprises a through cut (200), the through cut (200) defining an opening in the tubular wall, the through cut (200) having an edge configured to pierce barrier material (150) provided on needle assembly packaging (42) when the hub is depressed against the needle assembly packaging (42) to access a needle assembly (100) stored therein, and the opening of the through cut (200) being configured to receive the pierced barrier material (152) to guide the pierced barrier material (152) away from the needle assembly (100);
wherein the through cut (200) is disposed in the tubular wall at a designated angle (210) between a longitudinal axis of the pen body (4) and a perpendicular axis at the distal end (54);
wherein the designated angle (210) is selected to capture the barrier material (150) after being pierced by the edge of the through cut (200) and guide the barrier material (150) away from the needle assembly (100).

5. The adapter of claim 4, wherein the through cut (200) is disposed in the tubular wall at a designated angle (210) between the longitudinal axis of the hub and a perpendicular axis at the distal end of the hub.

6. The adapter of claim 5, wherein the designated angle (210) is selected to capture the barrier material (150) after being pierced by the edge of the through cut (200) and guide the barrier material (150) away from the needle assembly (100).

7. The adapter of claim 4, wherein the through cut (200) has a distal end adjacent the distal end (54) of the hub and a proximal end that is opposite the distal end of the through cut (200), and the opening extends between the distal end of the through cut (200) and the proximal end of the through cut (200); and
wherein the through cut (200) is arranged in a selected one of a first direction and a second direction, the first direction allowing the distal end of the through cut (200) to precede the proximal end of the through cut (200) when the hub is rotated in a clockwise direction about the longitudinal axis, and the second direction allowing the distal end of the through cut (200) to precede the proximal end of the through cut (200) when the hub is rotated in a counterclockwise direction about the longitudinal axis.

8. The adapter of claim 4, wherein the hub is dimensioned at its proximal end (51) to mate with a pen.

9. The adapter of claim 4, wherein the hub has a post section at its distal end (54) that is dimensioned to fit in a cavity (35)of the needle assembly packaging (42)in which a needle assembly (100) is stored and to receive at least part of the needle assembly (100); and
wherein the through cut (200) is disposed in the post section of the hub.

10. The adapter of claim 1 or 4, wherein the edge of the through cut (200) comprises a tooth (202) that extends from the distal end (54) of the hub and/or wherein the tooth extends from the distal end (54) of the hub by a distance corresponding to at least the thickness of the barrier material (150).

11. The adapter of claim 1 or 4, wherein the tooth (202) comprises a pointed or rounded tip.

12. The adapter of claim 1 or 4, wherein the edge of the through cut (200) comprises a scything edge (208) along at least one side of the opening.

13. The adapter of claim 1 or 4, wherein the opening of the through cut (200) has an arcuate or straight shape.

## Patentansprüche

1. Injektionspen, wobei der Injektionspen aufweist:
einen Pen-Körper (4), der eine rohrförmige Wand mit einem proximalen Ende (51) und einem entgegengesetzten distalen Ende (54) aufweist, das dazu ausgebildet ist, mit einer Nadelbaugruppe (100) des Injektionspens verbunden zu werden;
wobei das distale Ende (54) der rohrförmigen Wand einen Durchschnitt (200) aufweist, wobei der Durchschnitt (200) eine Öffnung in der rohrförmigen Wand definiert, wobei der Durchschnitt (200) einen Rand aufweist, der dazu ausgebildet ist, ein Barrierematerial (150) zu durchstechen, das auf einer Nadelbaugruppenverpackung (42) der Nadelbaugruppe (100) vorgesehen ist, wenn das distale Ende (54) gegen die Nadelbaugruppenverpackung (42) niedergedrückt wird, um auf die darin aufbewahrte Nadelbaugruppe (100) zuzugreifen, und wobei die Öffnung des Durchschnitts (200) so ausgebildet ist, dass sie das durchstochene Barrierematerial (152) aufnimmt, um das durchstochene Barrierematerial (152) von der Nadelbaugruppe (100) wegzuführen,
wobei der Durchschnitt (200) in der rohrförmigen Wand in einem bezeichneten Winkel (210) zwischen einer Längsachse des Pen-Körpers (4) und einer senkrechten Achse an dem distalen Ende (54) angeordnet ist;
wobei der bezeichnete Winkel (210) so ausgewählt wird, dass der Durchschnitt das Barrierematerial (150) erfasst, nachdem es von dem Rand des Durchschnitts (200) durchstochen wurde, und das erfasste Barrierematerial (150) von der Nadelbaugruppe (100) wegführt.

2. Injektionspen nach Anspruch 1, wobei der Durchschnitt (200) ein distales Ende angrenzend an das distale Ende (54) des Pen-Körpers (4) und ein proximales Ende aufweist, das entgegengesetzt zu dem distalen Ende des Durchschnitts (200) ist, und wobei sich die Öffnung zwischen dem distalen Ende des Durchschnitts (200) und dem proximalen Ende des Durchschnitts (200) erstreckt; und
wobei der Durchschnitt (200) in einer ausgewählten Richtung aus einer ersten Richtung und einer zweiten Richtung angeordnet ist, wobei die erste Richtung ermöglicht, dass das distale Ende des Durchschnitts (200) vor dem proximalen Ende des Durchschnitts (200) liegt, wenn der Pen im Uhrzeigersinn um seine Längsachse gedreht wird, und wobei die zweite Richtung ermöglicht, dass das distale Ende des Durchschnitts vor dem proximalen Ende des Durchschnitts (200) liegt, wenn der Pen gegen den Uhrzeigersinn um seine Längsachse gedreht wird.

3. Injektionspen nach Anspruch 1, wobei das distale Ende (54) so bemessen ist, dass es in einen Hohlraum (35) der Nadelbaugruppenverpackung (42) passt, in der eine Nadelbaugruppe (100) aufbewahrt wird, und mindestens einen Teil der Nadelbaugruppe (100) aufnimmt.

4. Adapter für einen Injektionspen, wobei der Adapter aufweist:
einen Ansatz, der eine rohrförmige Wand mit einem proximalen Ende (51), das zur Verbindung mit einem Injektionspen ausgebildet ist, und einem distalen Ende (54) aufweist, das zur Verbindung mit einer Nadelbaugruppe (100) ausgebildet ist, wobei die rohrförmige Wand einen Kanal darin definiert, der sich zwischen dem proximalen Ende (51) und dem distalen Ende (54) und entlang einer Längsachse des Ansatzes erstreckt, wobei der Kanal dazu ausgebildet ist, mindestens einen Teil der Nadelbaugruppe (100) darin aufzunehmen;
wobei das distale Ende (54) der rohrförmigen Wand einen Durchschnitt (200) aufweist, wobei der Durchschnitt (200) eine Öffnung in der rohrförmigen Wand definiert, wobei der Durchschnitt (200) einen Rand aufweist, der dazu ausgebildet ist, ein Barrierematerial (150) zu durchstechen, das auf einer Nadelbaugruppenverpackung (42) vorgesehen ist, wenn der Ansatz gegen die Nadelbaugruppenverpackung (42) niedergedrückt wird, um auf die darin aufbewahrte Nadelbaugruppe (100) zuzugreifen, und wobei die Öffnung des Durchschnitts (200) so ausgebildet ist, dass sie das durchstochene Barrierematerial (152) aufnimmt, um das durchstochene Barrierematerial (152) von der Nadelbaugruppe (100) wegzuführen,
wobei der Durchschnitt (200) in der rohrförmigen Wand in einem bezeichneten Winkel (210) zwischen einer Längsachse des Pen-Körpers (4) und einer senkrechten Achse an dem distalen Ende (54) angeordnet ist;
wobei der bezeichnete Winkel (210) so ausgewählt wird, dass das Barrierematerial (150) erfasst wird, nachdem es von dem Rand des Durchschnitts (200) durchstochen wurde, und das erfasste Barrierematerial (150) von der Nadelbaugruppe (100) weggeführt wird.

5. Adapter nach Anspruch 4, wobei der Durchschnitt (200) in der rohrförmigen Wand in einem bezeichneten Winkel (210) zwischen der Längsachse des Ansatzes und einer senkrechten Achse an dem distalen Ende des Ansatzes angeordnet ist.

6. Adapter nach Anspruch 5, wobei der bezeichnete Winkel (210) so gewählt wird, dass er das Barrierematerial (150) erfasst, nachdem es von dem Rand des Durchschnitts (200) durchstochen wurde, und das Führungsmaterial (150) von der Nadelbaugruppe (100) wegführt.

7. Adapter nach Anspruch 4, wobei der Durchschnitt (200) ein distales Ende angrenzend an das distale Ende (54) des Ansatzes und ein proximales Ende aufweist, das entgegengesetzt zu dem distalen Ende des Durchschnitts (200) ist, und wobei sich die Öffnung zwischen dem distalen Ende des Durchschnitts (200) und dem proximalen Ende des Durchschnitts (200) erstreckt; und wobei der Durchschnitt (200) in einer ausgewählten Richtung aus einer ersten Richtung und einer zweiten Richtung angeordnet ist, wobei die erste Richtung ermöglicht, dass das distale Ende des Durchschnitts (200) vor dem proximalen Ende des Durchschnitts (200) liegt, wenn der Ansatz im Uhrzeigersinn um die Längsachse gedreht wird, und wobei die zweite Richtung ermöglicht, dass das distale Ende des Durchschnitts (200) vor dem proximalen Ende des Durchschnitts (200) liegt, wenn der Ansatz gegen den Uhrzeigersinn um die Längsachse gedreht wird.

8. Adapter nach Anspruch 4, wobei der Ansatz an seinem proximalen Ende (51) so bemessen ist, dass er mit einem Pen zusammenpasst.

9. Adapter nach Anspruch 4, wobei der Ansatz an seinem distalen Ende (54) einen Säulenabschnitt aufweist, der so bemessen ist, dass er in einen Hohlraum (35) der Nadelbaugruppenverpackung (42) passt, in der eine Nadelbaugruppe (100) aufbewahrt wird, und mindestens einen Teil der Nadelbaugruppe (100) aufnimmt; und
wobei der Durchschnitt (200) in dem Säulenabschnitt des Ansatzes angeordnet ist.

10. Adapter nach Anspruch 1 oder 4, wobei der Rand des Durchschnitts (200) einen Zahn (202) aufweist, der sich von dem distalen Ende (54) des Ansatzes aus erstreckt und/oder wobei sich der Zahn von dem distalen Ende (54) des Ansatzes um einen Abstand erstreckt, der zumindest der Dicke des Barrierematerials (150) entspricht.

11. Adapter nach Anspruch 1 oder 4, wobei der Zahn (202) eine zugespitzte oder abgerundete Spitze aufweist.

12. Adapter nach Anspruch 1 oder 4, wobei der Rand des Durchschnitts (200) einen Sensenrand (208) entlang mindestens einer Seite der Öffnung aufweist.

13. Adapter nach Anspruch 1 oder 4, wobei die Öffnung des Durchschnitts (200) eine bogenförmige oder gerade Form hat.

## Revendications

1. Stylo d'injection, le stylo d'injection comprenant :
un corps de stylo (4) comportant une paroi tubulaire avec une extrémité proximale (51) et une extrémité distale (54) opposée configurée pour être connectée à un ensemble aiguille (100) du stylo d'injection ;
l'extrémité distale (54) de la paroi tubulaire comprenant un évidement (200), l'évidement (200) définissant une ouverture dans la paroi tubulaire, l'évidement (200) ayant un bord configuré pour percer un matériau barrière (150) prévu sur un emballage d'ensemble aiguille (42) de l'ensemble aiguille (100) lorsque l'extrémité distale (54) est pressée contre l'emballage d'ensemble aiguille (42) pour accéder à l'ensemble aiguille (100) stocké là-dedans, et l'ouverture de l'évidement (200) étant configurée pour recevoir le matériau barrière percé (152) pour éloigner le matériau barrière percé (152) de l'ensemble aiguille (100),
l'évidement (200) étant disposé dans la paroi tubulaire à un angle désigné (210) entre un axe longitudinal du corps de stylo (4) et un axe perpendiculaire à l'extrémité distale (54) ;
l'angle désigné (210) étant choisi de façon que l'évidement capture le matériau barrière (150) après avoir été percé par le bord de l'évidement (200) et éloigne le matériau barrière (150) de l'ensemble aiguille (100).

2. Stylo d'injection selon la revendication 1, l'évidement (200) ayant une extrémité distale adjacente à l'extrémité distale (54) du corps de stylo (4) et une extrémité proximale qui est opposée à l'extrémité distale de l'évidement (200), et l'ouverture s'étendant entre l'extrémité distale de l'évidement (200) et l'extrémité proximale de l'évidement (200) ; et
l'évidement (200) étant agencé dans une direction choisie entre une première direction et une deuxième direction, la première direction permettant à l'extrémité distale de l'évidement (200) de précéder l'extrémité proximale de l'évidement (200) lorsque le stylo est tourné dans le sens des aiguilles d'une montre autour de son axe longitudinal, et la deuxième direction permettant à l'extrémité distale de l'évidement de précéder l'extrémité proximale de l'évidement (200) lorsque le stylo est tourné dans le sens inverse des aiguilles d'une montre autour de son axe longitudinal.

3. Stylo d'injection selon la revendication 1, l'extrémité distale (54) ayant des dimensions pour rentrer dans une cavité (35) de l'emballage d'ensemble aiguille (42) dans lequel l'ensemble aiguille (100) est stocké et pour recevoir au moins une partie de l'ensemble aiguille (100).

4. Adaptateur pour un stylo d'injection, l'adaptateur comprenant :
une pièce de connexion comprenant une paroi tubulaire ayant une extrémité proximale (51) configurée pour être connectée à un stylo d'injection et une extrémité distale (54) configurée pour être connectée à un ensemble aiguille (100), la paroi tubulaire définissant là-dedans un canal qui s'étend entre l'extrémité proximale (51) et l'extrémité distale (54) et le long d'un axe longitudinal de la pièce de connexion,
le canal étant configuré pour recevoir là-dedans au moins une partie de l'ensemble aiguille (100);
l'extrémité distale (54) de la paroi tubulaire comprenant un évidement (200), l'évidement (200) définissant une ouverture dans la paroi tubulaire, l'évidement (200) ayant un bord configuré pour percer un matériau barrière (150) appliqué sur un emballage d'ensemble aiguille (42) de l'ensemble aiguille (100) lorsque l'extrémité distale (54) est pressée contre l'emballage d'ensemble aiguille (42) pour accéder à l'ensemble aiguille (100) stocké là-dedans, et l'ouverture de l'évidement (200) étant configurée pour recevoir le matériau barrière percé (152) pour éloigner le matériau barrière percé (152) de l'ensemble aiguille (100),
l'évidement (200) étant disposé dans la paroi tubulaire à un angle désigné (210) entre un axe longitudinal du corps de stylo (4) et un axe perpendiculaire à l'extrémité distale (54) ;
l'angle désigné (210) étant choisi de façon que l'évidement capture le matériau barrière (150) après avoir été percé par le bord de l'évidement (200) et éloigne le matériau barrière (150) de l'ensemble aiguille (100).

5. Adaptateur selon la revendication 4, l'évidement (200) étant disposé dans la paroi tubulaire à un angle désigné (210) entre l'axe longitudinal de la pièce de connexion et un axe perpendiculaire à l'extrémité distale de la pièce de connexion.

6. Adaptateur selon la revendication 5, l'angle désigné (210) étant choisi de façon que l'évidement capture le matériau barrière (150) après avoir été percé par le bord de l'évidement (200) et éloigne le matériau barrière (150) de l'ensemble aiguille (100).

7. Adaptateur selon la revendication 4, l'évidement (200) ayant une extrémité distale adjacente à l'extrémité distale (54) de la pièce de connexion et une extrémité proximale qui est opposée à l'extrémité distale de l'évidement (200), et l'ouverture s'étendant entre l'extrémité distale de l'évidement (200) et l'extrémité proximale de l'évidement (200) ; et
l'évidement (200) étant agencé dans une direction choisie entre une première direction et une deuxième direction, la première direction permettant à l'extrémité distale de l'évidement (200) de précéder l'extrémité proximale de l'évidement (200) lorsque la pièce de connexion est tournée dans le sens des aiguilles d'une montre autour de son axe longitudinal, et la deuxième direction permettant à l'extrémité distale de l'évidement (200) de précéder l'extrémité proximale de l'évidement (200) lorsque la pièce de connexion est tournée dans le sens inverse des aiguilles d'une montre autour de son axe longitudinal.

8. Adaptateur selon la revendication 4, la pièce de connexion ayant des dimensions à son extrémité proximale (51) pour aller avec un stylo.

9. Adaptateur selon la revendication 4, la pièce de connexion ayant une partie tige à son extrémité distale (54) qui a des dimensions pour rentrer dans une cavité (35) de l'emballage d'ensemble aiguille (42) dans lequel un ensemble aiguille (100) est stocké et pour recevoir au moins une partie de l'ensemble aiguille (100) ; et l'évidement (200) étant disposé dans la partie tige de la pièce de connexion.

10. Adaptateur selon la revendication 1 ou 4, le bord de l'évidement (200) comprenant une dent (202) qui s'étend à partir de l'extrémité distale (54) de la pièce de connexion et/ou la dent s'étendant à partir de l'extrémité distale (54) de la pièce de connexion sur une distance correspondant au moins à l'épaisseur du matériau barrière (150).

11. Adaptateur selon la revendication 1 ou 4, la dent (202) comprenant une pointe pointue ou arrondie.

12. Adaptateur selon la revendication 1 ou 4, le bord de l'évidement (200) comprenant un bord coupant (208) le long au moins un côté de l'ouverture.

13. Adaptateur selon la revendication 1 ou 4, l'ouverture de l'évidement (200) ayant une forme arquée ou droite.
